Europäisches Patentamt

European Patent Office

Office européen des brevets

(19)

(11) Publication number: **0 261 955**
**A2**

(12) # EUROPEAN PATENT APPLICATION

(21) Application number: **87308430.5**

(22) Date of filing: **23.09.87**

(51) Int. Cl.⁴: **C 12 Q 1/68**
C 12 N 1/06, C 12 Q 1/34,
C 12 Q 1/36

(30) Priority: **26.09.86 US 911809**

(43) Date of publication of application:
**30.03.88 Bulletin 88/13**

(84) Designated Contracting States:
**DE FR GB IT NL SE**

(71) Applicant: **E.I. DU PONT DE NEMOURS AND COMPANY**
**1007 Market Street**
**Wilmington Delaware 19898 (US)**

(72) Inventor: **Hewitt, Peter Lloyd**
**11 Crescent Drive**
**Andover Massachusetts 01810 (US)**

(74) Representative: **Myerscough, Philip Boyd et al**
**J.A.Kemp & Co. 14, South Square Gray's Inn**
**London, WC1R 5EU (GB)**

(54) **Process for immobilization of DNA.**

(57) An improved procedure for isolating and immobilizing DNA utilizing a mixture of lytic enzymes, alkali metal hydroxide to denature DNA, and a chaotropic agent is provided. The process provides a simple, rapid and efficient method for obtaining single stranded DNA amenable to ready detection by hybridization assays.

EP 0 261 955 A2

**Description**

PROCESS FOR IMMOBILIZATION OF DNA

Technical Field

This invention relates to the field of DNA hybridization analysis and more particularly to the isolation of DNA for binding to a support and subsequent hybridization analysis.

Background Art

The so-called dot hybridization technique is potentially of great value in determining the presence of pathogenic organisms in clinical samples. In order to fulfill this potential, general procedures for lysing cells, immobilizing DNA and hybridizing probes to the DNA from many different types or organisms must be developed. As originally developed and currently used these procedures are not general, rather they are tailored to a known specific set of conditions for a particular organism. In the clinical setting if the conditions could be tailored for a particular organism, the test is unnecessary because the identity of the organism is already known. The procedures used must be robust allowing identification of a wide variety of microorganisms without assuming the organism is of one type or another.

The early dot hybridization procedures as represented by Kafatos et al. [Nucleic Acids Research, Volume 7(6), 1541-1552 (1979)] relied on standard DNA preparation techniques. Many of these are based upon the classical procedure of Marmur [Journal of Molecular Biology, Volume 3, 208-218 (1961)]. In general, lysis conditions were selected for the organism of interest, generally lysozyme treatment with or without detergent, detergent treatment alone, mechanical disruption or repeated freeze-thaw cycles. In some procedures, DNA was partially digested with restriction endonucleases and the protein content of the sample was minimized by protease digestion and deproteinization by phenol extraction. Before the DNA could be bound to the hybridization membrane, it had to be rendered single stranded. This could be done either by heating to about 95°C or with high pH solutions. Both procedures have disadvantages. Heat denaturation requires that the processed samples be filtered through the hybridization membrane hot to avoid cooling and renaturing back to the double stranded form. Alkaline denaturation requires neutralization in order to achieve maximal binding of the DNA to the membrane.

Later development of the so-called quick-blot procedures [Bresser et al., DNA, Volume 2(3), 243-254 (1983)] eliminated some of the more tedious processing steps such as the deproteinization by organic extraction. These proicedures still used the traditional lysing procedures, such as lysozyme digestion, which are not effective on a variety of clinically significant organisms. These procedures use chaotropic salts, particularly sodium iodide, to assist in obtaining good binding of DNA to the hybridization membrane. This use of chaotropic salts; however, called for high concentrations of said salts, such as the 12.2 molal sodium iodide used by Bresser et al. Use of that high a concentration of salt has the disadvantage of requiring at least one washing step, Bresser et al. use a 70% ethanol wash, to remove excess salt.

Groet et al., United States Patent 4,588,682, issued May 13, 1986, disclose a method of binding nucleic acid to a support using a liquid binding solution to treat the nucleic acid which had been denatured after it had been deposited on the support. The binding solution, usually an organic solvent, is required to be a non-solvent for the support. This method does not distinguish between sample nucleic acid as is and purified nucleic acid.

Marmur discloses one of the traditional procedures for lysing microorganisms for the purpose of isolating DNA from them. In this procedure, a cell suspension is first treated with a detergent, sodium dodecyl sulfate, at 60°C in an attempt to lyse the cells. If lysis is achieved, one proceeds to the next step; if not, an enzyme, lysozyme, is added and allowed to digest the cells for 30-60 minutes. These steps can be reversed but, if they are, the detergent must be added for the subsequent processes to work correctly. This method of cell lysis, while broadly effective, is not universally so. There are oganisms with cell walls which are resistant to both lysozyme and detergent treatment (e.g., Streptococcus pyogenes). According to Marmur, it would be very difficult to devise a technique for the efficient isolation of DNA from a wide variety of microorganisms. Marmur's method is effective against various organisms including almost all Gram negative organisms and many Gram positive organisms. One organism of great interest not lysed by this method is Streptococcus pyogenes. This organism is the causative agent for the common illness, strep throat.

Gross-Bellard et al. [European Journal of Biochemistry, Volume 36, 32-38 (1973)] disclose a method for lysing mammalian cells. They report the use of Proteinase K and a detergent, sodium dodecyl sulfate (SDS), to lyse the cells. This method is also applicable to certain microorganisms susceptible to these lysis conditions.

Chassy et al. [Applied and Environmental Microbiology, Volume 39(1), 153-158 (1980)], disclose a procedure for extending the usefulness of lysozyme in lysing microorganisms. This procedure depends upon growing the organism in a modified medium, particularly one containing L-threonine. This leads to organisms with weakened cell wall crosslinks which are susceptible to lysozyme treatment. This procedure is useful only when the organism to be lysed is known to grow in the modified medium and precludes any possibility of using DNA collected directly from a clinical specimen without culturing said organism. therefore, this procedure is not of general utility.

De Klowet [Journal of Mircobiological Methods, Volume 2, 189-196 (1984)] discloses a method for rapid isolation of high molecular weight RNA and DNA from yeast through the use of a glucanase enzyme, lyticase,

isolated from <u>Oerskovia xanthineolytica</u> in the presence or absence of sodium dodecyl sulfate to lyse the cells.

Monsen et al. [FEMS Microbiology Letters, Volume 16, 19-24 (1983)] disclose a general method for cell lysis and preparation of DNA from streptococci. This method uses the lytic enzyme mutanolysin (endo-N-acetylmuraminidase) isolated from <u>Streptomyces globisporus</u> 1829 to lyse the organism. As noted above, these organisms are resistant to lysozyme and detergent induced lysis. Monsen et al. also showed that streptococci are reissant to a general protease, Proteinase K.

None of the known methods offers a completely general method for obtaining DNA bound to a support for hybridization through lysis of microorganisms, rendering the DNA single stranded and immobilization.

Disclosure of the Invention

A method of preparing DNA bound to a support for hybridization analysis comprising:

(A) forming a suspension of source organisms suspected of containing the DNA of interest;

(B) treating said organisms with at least one lytic enzyme;

(C) degrading proteins in the suspension by digestion with at least one broadly active protease;

(D) denaturing said DNA by treatment with alkali metal hydroxide;

(E) treating the mixture resulting from step (D) with at least one chaotropic agent; and

(F) immobilizing the single-stranded DNA on a support by contacting the mixture resulting from step (E) with said support.

Description of the Invention

To meet the need for a general method of obtaining DNA bound to a support from a variety of sources, the new process should provide broad spectrum lysis and rapid, high yield recovery and efficient immobilization of DNA from a wide variety of source organisms. By source organisms is meant any organism which contains DNA including cells, particularly microbial cells, viruses, and mycoplasma. The cells subjected to this process include cells of mammalian or bacterial origin or a mixture of mammalian and bacterial cells. Surprisingly, it has been found that the method of this invention allows isolation of DNA from a very wide variety of organisms in approximately 20 minutes. The single-stranded DNA so isolated can be immobilized on a support ready for hybridization analysis.

For sake of convenience, the process will be described as being carried out with a suspension of cells but it should be understood that other DNA containing source organisms can be treated similarly. As a practical matter, it is convenient to begin this process with a sample cell suspension containing $1 \times 10^5$ to $1 \times 10^6$ cells in 100 μL buffer. A variety of buffers can be used, including sodium borate or sodium phosphate, but tris(hydroxymethyl)aminomethane hydrochloride (Tris) is preferred. The buffer concentration can be about 1-500 mM with 10 mM being preferred. The preferred pH is about 8 although a pH ranging from about 4 to about 9 is acceptable. The buffer can also contain about 1-500 mM sodium chloride with 10 mM being preferred and, similarly about 1-10 mM ethylenediaminetetraacetic acid (EDTA) with about 1 mM being preferred. The preferred buffer composition is 10 mM Tris, 10 mM sodium chloride, 1 mM EDTA, pH 8.0 (Tris buffer, pH 8.0). The process can be readily adapted to accommodate larger volumes and/or different numbers of cells by adjusting dilutions and process timing.

The method of this invention can be carried out using a single lytic enzyme but is preferred that a mixture of such enzymes be used. The mixture of lytic enzymes to be added to the organism suspension is generally a bacteriolytic enzyme "cocktail" and can include lysozyme, endo-N-acetylmuraminidase and achromopeptidase among others. The final concentrations of the enzymes in the lytic mixture can be in the range of 50-500 μg/mL, 10-500 μg/mL and 50-500 μg/mL, respectively. The preferred concentrations are 300 μg/mL, 30 μg/mL and 300 μg/mL, respectively.

This lytic enzyme cocktail can be prepared in the Tris buffer, pH 8.0 described above, or any of the generally acceptable buffers can be utilized. This cocktail can be added to the suspension described above at a temperature range of about 20°C-70°C and incubated for a time period of about 1-60 minutes. It is known that, in general, enzymatic processes proceed more rapidly at higher tempeatures provided the enzyme is not denatured by the higher temperature. It is thus preferred to operate at the highest possible non-denaturing temperature for a given enzyme cocktail in order to minimize the time required to complete digestion. This same consideration will apply to all subsequent enzymatic degradation steps. The preferred treatment conditions for the above enzyme mixture are a digestion temperature of about 37°C for about 5 minutes. Optionally, a reducing agent can be added to the lytic mixture to further improve cell lysis. A variety of reducing agents can be used including dithiothreitol, dithioerythritol, cysteine and ascorbic acid, 2-mercaptoethanol being preferred. The final concentrations of any of the reducing agents can be in the range of 0.1-30 mM. The preferred concentration is 5 mM. To further improve both cell lysis and solubilization, aprotic solvent can be added to the lytic mixture. Among such solvents are N,N-dimethylformamide (DMF) and sulfolane with dimethylsulfoxide (DMSO) being a preferred one. The final concentration of the aprotic solvent can be in the range of 1-20% (v/v). The preferred concentration is 10% (v/v).

The lytic effect of this particular enzyme mixture is broad and includes microorganisms that can be found in the following genera:

<u>Aerobacter</u> sp.

<u>Acholeplasma</u> sp.

<u>Achromobacter</u> sp.

Arthrobacter sp.
Azotobacter sp.
Bacillus sp.
Blevibacterium sp.
Clostridium sp.
Enterobacter sp.
Escherichia sp.
Flavobacterium sp.
Klebsielle sp.
Kurthia sp.
Lactobacillus sp.
Leuconostoc sp.
Microccocus sp.
Mycoplasma sp.
Pediococcus sp.
Proteus sp.
Protaminobacter sp.
Pseudomonas sp.
Salmonella sp.
Sarcina sp.
Serratia sp.
Shigella sp.
Staphylococcus sp.
Streptococcus sp.
Streptomyces sp.

Other enzymes can also be added to the above enzyme cocktail to further broaden its bacteriolytic action. Suitable enzymes include: lipase, lysopeptase, endo-N-acetylglucosaminidase D or H, dextranase, glucoamylase, cellulase, N-acetylmuramyl-L-alanine amidase, hyaluronidase, streptomyces KM endopeptidase, streptomyces SA endopeptidase and streptomyces ML endopeptidase. Such enzymes will generally be useful in concentration ranges from about 10-500 µg/mL of the lytic mixture. Selection of one or more additional enzymes for inclusion in the lytic cocktail will depend upon the cell wall and cell membrane structure of the cell to be lysed. For example, the streptomyces ML endopeptidase can be added to improve lysis of cells containing type III peptidoglycans. The enzymatic specificity of each of these enzymes is generally known and enzyme selection is expected to be based on the type of organism present.

The cellular proteins present in the lytic mixture can then be degraded by treatment with a non-specific protease or a mixture of such broadly active proteases. The preferred protease is Proteinase K, but others such as a protease isolated from Streptomyces griseus [Pronase® (Calbiochem-Behring)] can be substituted. The final concentration is preferably about 50-500 µg/mL for about 5-60 minutes at about 20-70°C. A preferred specific treatment condition is approximately 300 µg/mL of Proteinase K at approximately 60°C for approximately 5 minutes. Optionally, a reducing agent can be added to the lytic mixture to further improve the protein degradation. A variety of reducing agents can be used including dithiothreitol, dithioerythritol, cysteine and ascorbic acid, 2-mercaptoethanol being preferred. The final concentrations of any of the reducing agents can be in the range of 1-100 mM. The preferred concentration is 5 mM. To further improve both cell lysis and solubilization, aprotic solvent can be added to the lytic mixture. Among such solvents are N,N-dimethylformamide (DMF) and sulfolane with dimethylsulfoxide (DMSO) being a preferred one. The final concentration of the aprotic solvent can be in the range of 1-50% (v/v). The preferred concentration is 10% (v/v).

It is generally disadvantageous to treat the mixture with surfactants since these interfere with the binding of DNA to the supports used for hybridization analysis. If, however, a solution hybridization procedure were to be used, the addition of a surfactant can be desirable to aid solubilization of the cellular material and to minimize non-specific binding of the probe to the cellular debris. Among the many surfactants which could be used, sodium dodecyl sulfate is generally preferred. Useful surfactants include cationic, anionic and nonionic surfactants such as Triton X-100, octyl-β-D-glucopyranoside, 3-[(3-cholamidopropyl)dimethylammonio]-1-propane sulfonate (CHAPS) and 3-[(3-cholamidopropyl)-dimethylammonio]-2-hydroxy-1-propanesulfonate (CHAPSO). Effective concentrations are expected to range from about 0.05% to about 1% (w/v).

Surfactant treatment can be used in conjunction with hybridization on a support provided it is removed, for example by chromatography, prior to binding the DNA to the support. It is most advantageous to remove the surfactant immediately prior to immobilization of the DNA.

The processed sample is treated with an alkali metal hydroxide to denature DNA and to solubilize other cellular components. Sodium hydroxide, KOH or LiOH can be used, with NaOH being preferred, in a preferred concentration range from about 0.1 N to about 1 N. The time of treatment can range from about 1 minute to about 60 minutes and the temperature from about 20°C to about 90°C. A preferred specific treatment condition involves 0.4 N NaOH at 60°C for 10 minutes. Heating accelerates the solubilization process which, in the case of RNA, is hydrolysis. It is expected that proteins and, to a lesser extent, DNA can also be hydrolyzed at higher base concentration and/or longer treatment times. Most advantageously, treatment with alkali metal hydroxide is carried out under conditions which would lead to sufficient hydrolysis of RNA and proteins and substantially no hydrolysis of DNA. Optionally, an alkaline protease, such as that isolated from Streptomyces griseus, can also be added to further degrade proteins.

The sample so processed is further treated with a chaotropic agent or a mixture of chaotropic agents to denature and dissociate from DNA any remaining proteins and to facilitate the binding of DNA to a hybridization support. By chaotropic agent is meant any substance capable of altering the secondary and tertiary structure of proteins and nucleic acids. Although the mechanism of the action of the chaotropic agent is not known with certainty, it is believed that the chaotrope adjusts the solution properties to allow both the efficient binding of DNA to the support and the availability of bound DNA for subsequent hybridization. The preferred chaotropic agents are salts such as ammonium acetate, sodium perchlorate, and sodium iodide. The preferred salt is sodium trifluoroacetate. Salt concentration can range from about 0.1 M-2 M; the temperature of treatment from aobut 20-90°C; and the time of treatment from about 1-30 minutes. It is most preferred to use 1 M sodium trifluoroacetate for about 5 minutes at 60°C.

A variety of supports can be utilized in the method of this invention; these are the supports generally utilized in DNA hybridization assays. Membranes are a preferred form of support; a charge-modified nylon membrane is most preferred. DNA can be bound to a membrane for hybridization analysis by any of the known methods.

The following Examples illustrate the invention.

EXAMPLE 1

ISOLATION AND IMMOBILIZATION OF DNA

A. Sample Preparation

A series of samples of Vero cells (ATCC Accession No. CCL 81) infected with Mycoplasma salivarium (ATCC Accession No. 23064) was obtained from Bionique Laboratories, Inc. (Saranac Lake, NY). These samples contained $10^{10}$, $10^9$, $10^8$, $10^7$, $10^6$ and $10^5$ Mycoplasma salivarium cells each in $10^5$ Vero cells/mL of sample suspension and were stored at -70°C.

A similar series of Samples of Acholeplasma laidlawii (ATCC Accession number 23206)-infected Vero cells was also obtained from Bionique Laboratories, Inc. These samples contained $10^{10}$, $10^9$, $10^8$, $10^7$, $10^6$ and $10^5$ Acholeplasma laidlawii cells each in $10^7$ Vero cells/mL of sample suspension and were stored at -70°C. For use, these samples were thawed, diluted 1:10 in 10 mM Tris buffer, pH 8.0, containing 10 mM NaCl and 1 mM EDTA (TE buffer).

B. Cell Lysis and DNA Preparation

A lytic enzyme cocktail was prepared by combining 150 μL of lysozyme (10 mg/mL) in water, 150 μL of achromopeptidase (10 mg/mL) in water, and 150 μL of endo-N-acetylmuramidase (1 mg/mL) in water. This cocktail was stored frozen at -20°C.

The lytic enzyme cocktail was thawed and 45 μL was added to 500 μL of a thawed cell suspension of $10^{10}$ Mycoplasma salivarium cells in Vero cells. The mixture was vortexed and incubated at 37°C for 10 minutes.

20 μL of 10 mg/mL Proteinase K was added to the sample, vortexed and incubated at 60°C for 5 minutes.

50 μL of 5 N sodium hydroxide was added to the sample, vortexed and incubated at 60°C for 10 minutes.

615 μL of 2 M sodium trifluoroacetate that was first filtered with a 0.22 μm Nalgene filter was added to the sample. The sample was vortexed and set aside at room temperature for about 5 minutes.

The remaining Mycoplasma salivarium and Acholeplasma laidlawii cells samples were processed in the same manner.

C. Immobilization of the DNA onto a Membrane

A Schleicher & Schuell (S & S) Minifold II Slot-Blotter was assembled with 1 sheet of GeneScreen Plus® membrane (available from E. I. du Pont de Nemours and Company) cut to fit into the Slot-Blotter was pre-wetted in sterile, deionized water and 2 sheets of S & S GB003 blotter paper that had also been pre-wetted in water.

A vacuum of 15 inches of mercury was applied to the Slot-Blotter to load the single stranded DNA samples into sample wells by filtration. 200 μL of each processed sample was filtered through different positions of the membrane with the Slot-Blotter as follows: 100 μL of 1 M sodium trifluoroacetate was applied to a sample well to wet the membrane and allowed to pass completely through the membrane followed by 200 μL of a sample and finally by 100 μL of 1 M sodium trifluoroacetate. Control samples of unlabeled probe DNA (described below) containing $1 \times 10^4$-$1 \times 10^8$ DNA molecules/200μL were similarly immobilized. Afterwards, the membrane was removed from the Slot-Blotter and air-dried for 30 minutes at room temperature.

D. DNA Probe Preparation

Plasmid DNA [pKK3535, Brosius et al., Plasmid, Volume 6, 112-118 (1981)], known to contain DNA sequences which are capable of hybridizing with those portions of the genomic DNA which code for r(RNA), was labeled with $^{32}$p for use as a DNA probe using a Nick-translation Kit (available from E. I. du Pont de Nemours and Company). The following were added to a 1.5-mL Eppendorf tube: 9 μL of 550 μg/mL plasmid DNA; 61 μL of water, 50 μL of nick-translation buffer; 40 μL of a solution containing dATP, dGTP, and dTTP; 1 millicurie of $^{32}$P-dCTP with a specific activity of 3000 Curies/mmole in a volume of 100 μL; 20 μL of DNA

polymerase I; and 20 µL of DNase I. The contents were mixed by drawing them into a Rainin P-200 pipet tip and expelling them back into the tube. The reaction was then allowed to proceed at 15°C for 60 minutes. The reaction was stopped with the addition of 6 µL of 500 mM EDTA. The $^{32}$P-labeled DNA probe was then separated from the unincorporated deoxynucleotide triphosphates by size separation on a 0.7 x 30 cm Sephadex G-50 column with TE buffer, pH 8.0. The specific activity of the labeled double stranded DNA probe was approximately 3 x 10$^8$ dpm/µg DNA.

### E. DNA Probe Hybridization

The hyridization membrane containing the immobilized DNA samples was pre-hybridized with 100 µg/mL sonicated, denatured, salmon sperm DNA for 30 minutes in 200 mL of 3X SSC buffer (3X SSC buffer contains 0.45 M NaCl and 0.045 M sodium citrate), containing 0.5% sodium dodecylsulfate (SDS) and 10X Denhardt's solution (10X Denhardt's solution contains 0.2% Ficoll, 0.2% polyvinylpyrolidone and 0.2% bovine serum albumin, fraction 5), at 68°C in a sealed plastic box on a rocker platform. The purified $^{32}$P-labeled DNA was then denatured by heating it to 100°C in a boiling water bath for 5 minutes, quickly cooled to 4°C on ice, and added immediately to the pre-hybridization mix on the membrane to achieve approximately 4 x 10$^6$ dpm/mL. Hybridization was allowed to proceed for 20 hours at 68°C with continuous rocking. After 20 hours, any unreacted $^{32}$P-labeled DNA probe was removed by four successive 15-minute washes with 100 mL of warm (68°C) 3X SSC buffer containing 0.5% SDS with continuous rocking. The membrane was then removed from the wash solution and air dried at room temperature.

### F. Detection of Hybridization

Bacterial DNA in the presence of the mammalian DNA from Vero cells was detected by visualization of the labeled hybrids by autoradiography of the membrane on x-ray film. The lower limit of detection for both Mycoplasma salivarium and Acholeplasma laidlawii cells was 1 x 10$^7$ bacteria/mL of suspension in the presence of Vero cells. There was no detectable cross-reactivity of the probe DNA with the mammalian DNA. The detection of 1 x 10$^7$ bacteria/mL sample represents a sensitivity of 1 x 10$^5$ molecules of target DNA. This Example demonstrates that the process of this invention permits simple, rapid and efficient isolation and immobilization of single-stranded DNA which can be detected in a hybridization assay.

### EXAMPLE 2

## ISOLATION AND IMMOBILIZATION OF DNA FROM URINE

### A. Sample Preparation

To 1 mL of a urine sample in a 1.5-mL Eppendorf tube were added approximately 1 x 10$^6$ human embryonic lung (HEL) cells infected with cytomegalo-virus (CMV) AD169. The sample was vortexed and then centrifuged at approximately 13,000 x g for 15 minutes at room temperature. After centrifugation, the supernatant was discarded. 50 µL of TE buffer was added to the tube and vortexed to resuspend the infected cells.

### B. Cell Lysis and DNA Preparation

The resuspended cells were lysed and DNA prepared for immobilization as described in Example 1(B) with the following changes: 20 µL of 5 mg/mL Proteinase K was added instead of 20 µL of 10 mg/mL concentration and the sample was incubated for 15 minutes. 10 µL of 5 N sodium hydroxide was added instead of 50 µL and incubated for 15 minutes. 120 µL of 2 M sodium trifluoroacetate was used rather than 615 µL.

### C. Immobilization of the DNA onto a Membrane

Immobilization was carried out as described in Example 1(C), however after DNA immobilization, the membrane was heated in a vacuum oven at 70°C for 90 minutes.

### D. Preparation of Labeled DNA

The BamHI 9.3-kilobase J fragment of CMV AD169 [Greenway et al., Gene, Volume 18, 355-360 (1982)] was labeled with $^{32}$P for use as a DNA probe using a Nick-translation Kit (available from E. I. du Pont de Nemours and Company). The following were added to a 1.5-mL Eppendorf tube: 20 µL of the DNA fragment in 10 mM Tris buffer, pH 7.0, containing 1 mM EDTA; 50 µL of water, 50 µL of nick-translation buffer; 40 µL of a solution containing the deoxynucleotide triphosphates dATP, dGTP and dTTP; 1 millicurie of $^{32}$P-dCTP with a specific activity of 3000 Curies/mmole in a volume of 100 µL; 20 µL of DNA polymerase I; and 20 µL of DNase I. The contents were mixed by drawing them into a Rainin P-200 pipet tip and expelling them back into the tube. The reaction was then allowed to proceed at 15°C for 60 minutes. The reaction was stopped with the addition of 50 µL of 250 mM EDTA. The $^{32}$P-labeled DNA was then separated from the unincorporated deoxynucleotide triphosphates as follows: the reaction mixture was transferred with a Rainin P-1000 pipet tip from the 1.5-mL Eppendorf tube to the top of a NENsorb℗ column bed (available from E. I. du Pont de Nemours and Company). The column was then washed twice with 3 mL of 10 mM Tris buffer, pH 7.0, containing 1 mM EDTA to remove any unincorporated deoxynucleotide triphosphates. The pure $^{32}$P-labeled DNA probe was eluted from the column with 1 mL of 20% ethanol. The specific activity of the labeled DNA was approximately 3 x 10$^8$ dpm/µg DNA.

E. DNA Probe Hybridization

The hybridization membrane containing the immobilized DNA samples was prehybridized for 60 minutes at 70°C in 10 mL of prehybridization buffer (5X SSPE buffer, 5X Denhardt's solution, 0.5% SDS and 200 μg/mL sonicated denatured salmon sperm DNA in water). 5X SSPE buffer contains 50 mM sodium phosphate buffer, pH 7.4, 0.9 M NaCl and 5 mM EDTA. Labeled double stranded DNA containing approximately 1 x $10^8$ dpm of radiolabel was denatured by first placing it in a boiling water bath at 100°C for 5 minutes and quickly cooling it to 4°C on ice. The immobilized DNA was hybridized with 1 x $10^7$ dpm/mL of $^{32}$P-labeled DNA probe in 10 mL of prehybridization buffer for 20 hours with continuous agitation at 70°C.

After 20 hours, any unreacted $^{32}$P-labeled DNA probe was removed by successive washes in a plastic box at 65°C as follows and air dried at room temperature: 400 mL of 1X SSPE buffer, pH 7.4, containing 0.1% SDS, for 10 minutes with agitation followed by two portions of 400 mL of 0.1% SDS for 30 minutes and 10 minutes, respectively, with agitation.

F. Detection of Hybridization

The target DNA from the HEL cells infected with CMV was detected by visualization of the labeled hybrids by autoradiography of the membrane on x-ray film. The results showed that the method of this invention permits isolation and immobilization of target DNA which can then be detected in a hybridization assay.

EXAMPLE 3

ISOLATION AND IMMOBILIZATION OF DNA FROM BACTERIA

A. Sample Preparation

The organisms listed in Table 1 were grown in Petri dishes overnight at 37°C on blood agar base medium.

## TABLE 1

### ORGANISMS USED

| Organism | Du Pont Strain Number | ATCC Accession Number | Nomenclature Status |
|---|---|---|---|
| Bacillus subtilis | 379 | 6051 | Type |
| Enterobacter chloacae | 375 | 13047 | Type |
| Escherichia coli | 282 | 11775 | Neotype |
| Klebsiella pneumoniae | 373 | 13883 | Type |
| Proteus mirabilis | 374 | 29906 | Type |
| Pseudomonas aeruginosa | 295 | 10145 | Neotype |
| Staphylococcus aureu | 381 | 12600 | Neotype |
| Staphylococcus epidermidis | 382 | 14990 | Neotype |
| Serratia marcescens | 372 | 13880 | Type |
| Streptococcus faecalis | 283 | 19433 | Type |

Several single colonies were removed from each of the Petri dishes with a pasteur pipet, suspended in 1 mL of TE buffer, and vortexed in 12 x 75 mm borosilicate glass test tubes. A concentration of approximately $3 \times 10^8$ cells/mL was achieved by diluting each of the cell suspensions with the TE buffer. The cell concentrations were determined by a visual comparison of each of the cell suspensions with a McFarland nephelometric turbidity standard of 1 in a 12 x 75 mm borosilicate glass test tube. 500 µL of each of the cell suspensions was then transferred to another 12 x 75 mm borosilicate glass test tube for further processing.

B. Cell Lysis and DNA Preparation
The cells were lysed and DNA prepared for immobilization as described in Example 1(B).

C. Immobilization of the DNA onto a Membrane
Immobilization was carried out as described in Example 1(C).

D. DNA Probe Preparation
This probe was prepared as described in Example 1(D).

E. DNA Probe Hybridization
Hybridization was carried out as described in Example 1(E).

F. Detection of Hybridization
Target DNA from the various bacteria were detected as described in Example 1(F). The results showed that the method of this invention could be successfully applied to a wide variety of bacterial species to isolate and immobilize the target DNA for subsequent detection by hybridization assays.

**Claims**

1. A method of preparing DNA bound to a support for hybridization analysis comprising:
    (A) forming a suspension of source organisms suspected of containing the DNA of interest;
    (B) treating said organisms with at least one lytic enzyme;
    (C) degrading proteins in the suspension by digestion with at least one protease;
    (D) denaturing said DNA by treatment with alkali metal hydroxide;
    (E) treating the mixture resulting from step (D) with at least one chaotropic agent;
    (F) immobilizing the single-stranded DNA on a support by contacting the mixture resulting from step (E) with said support.
2. A method according to claim 1 wherein the source organism is selected from cells, viruses and mycoplasma.
3. A method according to claim 2 wherein said cells are microorganisms.
4. A method according to claim 1, 2 or 3 wherein the suspension of said organisms is treated with a mixture of lytic enzymes.
5. A method according to claim 4 wherein the mixture of lytic enzymes comprises lysozyme, endo-N-acetylmuraminidase and achromopeptidase.
6. A method according to any one of the preceding claims wherein the chaotropic agent is selected from sodium trifluoroacetate, sodium perchlorate and sodium iodide.
7. A method for isolating and immobilizing DNA from cells or viruses for hybridization analysis comprising:
    (A) forming a suspension of said cells or viruses containing the DNA of interest;
    (B) treating said cells or viruses in the suspension with a mixture of lytic enzymes comprising lysozyme, endo-N-acetylmuraminidase and achromopeptidase;
    (C) degrading proteins in the suspension by digestion with Proteinase K;
    (D) denaturing said DNA by treatment with alkali metal hydroxide;
    (E) treating the mixture resulting from step (D) with chaotropic salt; and
    (F) immobilizing the single stranded DNA on a membrane by contacting the mixture resulting from step (E) with said membrane.